# FASCICULE DE BREVET EUROPEEN

(11) **EP 1 458 347 B1**
(45) Date de publication et mention de la délivrance du brevet: **28.09.2016**
(21) Numéro de dépôt: 02799126.4
(22) Date de dépôt: 27.12.2002
(51) Int. Cl.: A61Q 19/00, A61K 8/68

(54) **COMPOSITION COMPRENANT AU MOINS UN ALCANOLAMIDE POUR INHIBER LA MIGRATION DES CELLULES DE LANGERHANS ET SES UTILISATIONS**
MINDESTENS EIN ALKANOLAMID ENTHALTENDE ZUSAMMENSETZUNG ZUR INHIBIERUNG DER MIGRATION DER LANGERHANSZELLEN UND DEREN VERWENDUNG
COMPOSITION COMPRISING AT LEAST AN ALKANOLAMIDE FOR INHIBITING LANGERHANS CELL MIGRATION, AND USES THEREOF

(30) Priorité: 27.12.2001 FR 0116916
(43) Date de publication de la demande: 22.09.2004
(73) Titulaire: Laboratoires Expanscience, 92400 Courbevoie (FR)
(72) Inventeur: MSIKA, Philippe, 78000 Versailles (FR); PICCARDI, Nathalie, 38120 Saint-Egreve (FR); PICCIRILLI, Antoine, 78000 Versailles (FR)
(74) Mandataire: Regimbeau
(86) Numéro de dépôt international: PCT/FR2002/004582
(87) Numéro de publication internationale: WO 2003/055462

(56) Documents cités:
- WO-A-95/29151
- WO-A-99/29293
- WO-A-99/41266
- WO-A-02/072082
- DE-A- 19 841 794
- US-A- 6 001 375
- US-A- 6 159 485
- E. BERARDESCA ET AL.: "Evaluation of efficacy of a skin lipid mixture in patients with irritant contact dermatitis, allergic contact dermatitis or atopic dermatitis: a multicenter study." CONTACT DERMATITIS, vol. 45, no. 5, 2001, pages 280-285, XP001128103 dk
- T. SAKAI ET AL.: "Ceramide derivatives as therapeutic agents" EXPERT OPINION ON THERAPEUTIC PATENTS, vol. 8, no. 12, 1998, pages 1673-1682, XP001127407
- G. HALLIDAY ET AL.: "Protein kinase C transduces the signal for Langerhans' cell migration from the epidermis." IMMUNOLOGY, vol. 79, 1993, pages 621-626, XP001011467
- R. ANORA ET AL: "Stimulation in vitro of galactocerebroside galactosidase by N-decanoyl 2-amino-2-methylpropanol" LIPIDS, vol. 7, no. 1, 1972, pages 56-59, XP000645675

## Description

La présente invention se rapporte au traitement cosmétique et pharmaceutique, notamment dermatologique de la peau. Plus particulièrement, la présente invention concerne une composition contenant au moins un composé actif choisi parmi les alcanolamides tels que définis dans les revendications, éventuellement en association avec au moins un autre composé tel qu'un inhibiteur de métalloprotéases, un inhibiteur de PKC, un agent anti-inflammatoire, un agent apaisant, un immunosuppresseur, un chélateur d'ions, une oxazolidinone, un dérivé d'acide carbamique ou une oxazoline pour son utilisation dans les traitements définis dans les revendications.

La publication « stimulation in vitro of galactocerebroside galactosidase by N-decanoyl 2-amino-2-methylpropanol », Arora et al., lipids vol.7, No. 1, 1972, décrit l'utilisation d'un alkanolamide particulier, le N-dodecanoyl 2-amino-2-méthyl propanol, pour inhiber la β-galactosidase dans le cerveau de rats. Cet article ne décrit pas le traitement des réactions ou pathologies de la peau et/ou des muqueuses consécutives à une migration des cellules de Langerhans.

La publication WO99/29293 décrit des compositions pour application topique comprenant une base libre sphingoïde et un céramique de formule différente des alcanolamides selon la présente invention puisqu'ils comprennent deux chaînes longues.

Le brevet US 6,001,375 décrit des compositions pour application topique de céramiques de structures différentes des alcanolamides selon la présente invention.

La publication « ceramide derivates as therapeutic agents », teruyuki Sakai et al., Exp. Opin. Ther. Patents (1998) décrit des dérivés de céramides, sphingosine, glycosyl céramides et sphingofungin.

L'invention a également pour objet une telle composition pour son utilisation comme médicament avantageusement pour inhiber la migration des cellules telles que les dendrocytes dermiques, les monocytes, les lymphocytes, et notamment les cellules de Langerhans, suite par exemple à un stimulus extérieur ou « signal danger » d'origine chimique, physique, biologique et plus particulièrement immunitaire, dont l'intensité serait suffisamment importante pour induire une perturbation de l'homéostasie cutanée, pour les traitements définis dans les revendications. Les alcanolamides tels que définis dans les revendications, ainsi que leur association avec un inhibiteur de métalloprotéases, un inhibiteur de PKC, un agent anti-inflammatoire, un agent apaisant, un immunosuppresseur, un chélateur d'ions, une oxazolidinone, un dérivé d'acide carbamique ou une oxazoline et les compositions pharmaceutiques les comprenant sont utiles pour la préparation de médicaments destinés au traitement ou à la prévention des pathologies cutanées d'origine allergique et/ou inflammatoire.

L'invention a également pour objet une composition telle que définie dans les revendications pour son utilisation dans une méthode de traitement des peaux et/ou des muqueuses sensibles, irritées, intolérantes, à tendance allergique, âgées, soumises à un signal danger, présentant un trouble de la barrière cutanée, présentant des rougeurs cutanées, ou présentant un trouble, un déséquilibre ou un désordre immunologique non pathologique, consistant à appliquer sur la peau et/ou les muqueuses une telle composition.

Une des principales fonctions de la peau est la protection de l'organisme contre des agressions du milieu extérieur. Cette protection est assurée en grande partie grâce à la coopération de cellules présentes dans la peau, cellules qui sont capables en présence d'un agent nocif de générer une réponse inflammatoire et/ou immunitaire dirigée contre l'agent nocif. Ce sont les cellules dendritiques, les cellules de Langerhans (CL) de l'épiderme, et dendrocytes dermiques, les monocytes, les lymphocytes, les kératinocytes, les mastocytes, et les cellules endothéliales vasculaires.

Les CL sont des cellules dendritiques issues de la moelle épinière et qui résident dans les tissus non lymphoïdes tels que la peau et les muqueuses (bouche, poumon, vessie, rectum, vagin). Dans la peau, les CL s'intercalent entre les kératinocytes épidermiques en position suprabasale. Sur le plan ultrastructural, elles sont caractérisées par la présence d'un organite spécifique d'origine membranaire, le granule de Birbeck. Sur le plan immunohistochimique, les CL expriment notamment la molécule CD1a et les molécules du Complexe Majeur d'Histocompatibilité de classe II.

Les CL jouent un rôle déterminant dans l'immunité, en tant que cellules présentatrices de l'antigène. En effet, des expériences menées chez la souris démontrent que les CL capturent les antigènes présents au niveau de l'épiderme et migrent vers les tissus lymphoïdes drainants la peau, où elles présentent l'antigène aux cellules T. L'initiation de la réponse immune cutanée dépend de la capacité des CL à quitter l'épiderme pour migrer jusqu'aux ganglions proximaux. Différents facteurs peuvent influencer cette migration : l'expression de molécules d'adhérence, les protéines de la matrice extracellulaire, des haptènes, des cytokines, etc. Néanmoins, les mécanismes impliqués dans la migration des CL ne sont pas totalement encore élucidés. En particulier, avant d'atteindre les ganglions lymphatiques, les CL doivent non seulement traverser la jonction dermo-épidermique (JDE) mais également se frayer un chemin au travers de la matrice extracellulaire (MEC) dermique. La JDE est principalement composés de laminine 5, de collagène de type IV et VII, de nidogène et de perlecan. La MEC qui entoure les fibroblastes du derme contient essentiellement des collagènes de type I et III.

La maturation, ainsi que l'initiation et la régulation de la migration des CL sont sous la dépendance de cytokines pro-inflammatoires telles que l'IL-1β (interleukine-1-beta) et le TNF-α (Tumor Necrosis-alpha). Il en résulte que toute agression cutanée, plus particulièrement toute réaction inflammatoire et/ou irritative, capable d'induire en quantité suffisante l'une ou l'autre de ces cytokines ou les deux, est capable de stimuler la migration des CL, et donc de faciliter la réaction allergique si ces CL sont associées à un antigène.

Des pathologies de type dermatologique peuvent être observées comme résultant de la migration des CL suite à la capture d'un antigène de surface. Dans l'eczéma atopique, les CL sont capables de fixer des IgE en surface et d'induire une réponse immunitaire pathologique. Dans l'eczéma de contact, les CL jouent un rôle central puisqu'elles captent et traitent l'antigène avant de le présenter aux lymphocytes T. Celui-ci va le garder en mémoire et la réaction immunitaire sera déclenchée au deuxième contact.

Compte tenu de ce qui précède, il est hautement souhaitable de pouvoir modifier la capacité migratoire des dendrocytes dermiques, des monocytes, des lymphocytes, des cellules de Langerhans (CL), pour tenter d'augmenter le seuil de tolérance ou de limiter la réactivité de la peau allergique et/ou inflammatoire et/ou irritée ainsi que de la peau atopique et/ou sensible et/ou réactive et/ou inconfortable. C'est le problème que se propose de résoudre la présente invention. Les inventeurs ont en effet démontré de manière tout à fait surprenante et inattendue que des composés tels que les alcanolamides définis dans les revendications permettent d'inhiber de manière spectaculaire la migration des cellules, telles que les cellules de Langerhans, notamment induite par la présence d'un agent allergène. Les alcanolamides définis dans les revendications n'ont jamais été décrits dans l'art antérieur comme capables d'inhiber la migration des cellules de Langerhans notamment induite par la présence d'un agent allergène.

La présente invention concerne ainsi l'utilisation d'une composition comprenant au moins un composé actif pour inhiber la migration des cellules de Langerhans choisi dans le groupe des alcanolamides définis dans les revendications pour l'obtention d'un médicament destiné au traitement ou à la prévention des réactions ou pathologies définis dans les revendications. Avantageusement, la composition est une composition cosmétique ou pharmaceutique, notamment dermatologique, comprenant au moins un excipient cosmétiquement ou pharmaceutiquement acceptable.

Les alcanolamides selon l'invention répondent aux formules générales suivantes: 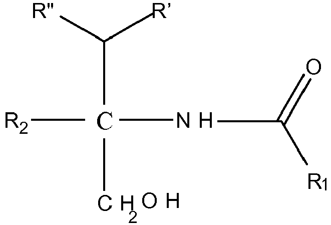 dans laquelle
le radical R₁ représente un groupe alkyle linéaire saturé comprenant de 2 à 40 atomes de carbone (en C₂-C₄₀), de manière avantageuse 6 à 22 atomes de carbone (en C₆-C₂₂), de manière encore plus avantageuse de 8 à 18 atomes de carbone (en C₈-C₁₈), et de manière encore plus avantageuse de 10 à 16 atomes de carbone (en C₁₀-C₁₆). Dans un mode de réalisation préféré de l'invention, R₁ représente un groupe alkyle linéaire saturé en C₁₁.

R' et R" représentent, de manière indépendante, un atome d'hydrogène, un groupe méthyle ou un groupe alkyle linéaire saturé en C₂-C₂₀.

R₂ représente un atome d'hydrogène, un groupe méthyle ou un groupe alkyle linéaire saturé en C₂-C₂₀.

Selon la présente invention, le radical R₁ représente un groupe alkyle linéaire saturé en C₂-C₄₀, avantageusement en C₆-C₂₂, encore plus avantageusement en C₈-C₁₈ et de manière encore plus avantageuse en C₁₀-C₁₆ et R' et R" représentent, de manière indépendante, un atome d'hydrogène, un groupe méthyle ou un groupe alkyle linéaire saturé en C₂-C₂₀ et R₂ représente un atome d'hydrogène, un groupe méthyle ou un groupe alkyle linéaire saturé en C₂-C₂₀.

Selon une variante avantageuse de l'invention, le dit alcanolamide est l'alcanolamide appelé AK100 de formule :

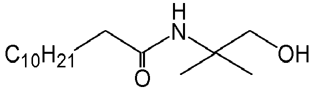

Selon un mode de réalisation de l'invention, la composition peut comprendre en outre au moins un inhibiteur de la migration des cellules de Langerhans sélectionné dans le groupe composé des inhibiteurs de métalloprotéases matricielles (MMPs).

Par « composés inhibiteurs des métalloprotéases matricielles (MMPs) » on entend selon l'invention tout composé connu de l'homme du métier pour sa capacité d'inhiber l'activité de dégradation de la matrice extracellulaire par les MMPs. Les MMPs constituent une famille d'enzymes (actuellement plus d'une vingtaine ont été identifiées et caractérisées), zinc-dépendantes, de structure très conservée, qui possèdent la capacité de dégrader les composants de la matrice extracellulaire. Elles sont classées selon la nature de leur substrat en collagénases, gélatinases et stromélysine. Elles peuvent être synthétisées par différents types cellulaires au niveau de la peau (fibroblastes, kératinocytes, macrophages, cellules endothéliales, éosinophiles, cellules de Langerhans, etc.). Le groupe des MMPs est ainsi constitué de quatre sous-classes : (1) les collagénases, (2) les gélatinases, (3) les stromelysines et (4) les MMP de type membranaires (MT-MMPs). L'activité des MMPs peut être modulée par des inhibiteurs de protéinase naturellement présents tels que les inhibiteurs tissulaires de métalloprotéinases (TIMPs ; notamment les TIMP-1 et TIMP-2). En particulier, le composé actif pour inhiber la migration des cellules de Langerhans est un composé inhibiteur d'au moins une MMP choisie dans le groupe constitué par les MMP-1, MMP-2, MMP-3 MMP-9, MMP-13 et MMP-18. Comme « composé inhibiteur des MMPs », en tant que composé actif pour inhiber la migration des cellules de Langerhans selon la présente invention, on entend en particulier les inhibiteurs tissulaires de métalloprotéinases (TIMPs), l'alpha-2-macroglobuline, les inhibiteurs de l'activateur du plasminogène, les chélateurs de zinc, la bryostatine-1, les antibiotiques (doxycyclines, minocyclines, etc.), les peptides synthétiques ou naturels ayant une structure similaire aux substrats des MMPs (batimastat, marimastat, etc.), les rétinoïdes (en particulier les rétinoïdes non aromatiques tels le rétinaldéhyde, la trétinoïne, et l'acide rétinoïque 9-cis, la vitamine A, les rétinoïdes monoaromatiques tels l'étrétinate, l'all-trans acitretine et le motrerinide, et les rétinoïdes polyaromatiques tels que l'adapalène, le tazarotène, le tamibarotène et l'arotinoïde methyl sulfone), les antioxydants (les piègeurs d'oxygène singulet, etc.), les anti-cancéreux (ou « anti-métastatiques »), les hydrolysats de malt tels que Colalift commercialisés par la société Coletica, les extraits d'algues marines tels que Kelpadélie commercialisés par la société Secma, les extraits de cartilage de requin tels que le complexe MDI commercialisés par la société Atrium, les peptides de riz comme par exemple Colhibin commercialisé par la société Pentapharm, les extraits peptidiques de lupin. Plus particulièrement, le composé inhibiteur des MMPs selon la présente invention est choisi dans le groupe constitué par les extraits peptidiques de lupin ou « peptides de lupin », tels que ceux décrits dans la demande de brevet FR-99 04 875 déposée le 19 avril 1999 au nom de la société Laboratoires Pharmascience. On peut notamment citer l'extrait peptidique décrit dans la demande FR 99 04875 sous la dénomination extrait B (LU105). Selon un autre mode préféré de réalisation, le dit inhibiteur des MMPs est choisi dans le groupe constitué par les rétinoïdes.

Selon un mode de réalisation particulier de l'invention, la composition peut également comprendre en outre au moins un composé choisi dans le groupe constitué par les inhibiteurs de PKC, les agents anti-inflammatoires, les agents apaisants, les immunosuppresseurs, les chélateurs d'ions, les oxazolidinones, les dérivés d'acide carbamique, notamment le (1-Hydroxyméthyl-tridécyl)-acide carbamique et le (1-Hydroxyméthyl-undécyl)-acide carbamique, et les oxazolines. Ce ou ces composé(s) choisi(s) dans le groupe constitué par les inhibiteurs de PKC, les agents anti-inflammatoires, les agents apaisants, les immunosuppresseurs, les chélateurs d'ions, les oxazolidinones, les dérivés d'acide carbamique, notamment le (1-Hydroxyméthyl-tridécyl)-acide carbamique et le (1-Hydroxyméthyl-undécyl)-acide carbamique, et les oxazolines permet(tent) de jouer sur et/ou de limiter la réaction irritative ou de sensibilisation voire pour certains d'entre eux également d'inhiber la migration des cellules dendritiques, plus particulièrement des cellules de Langerhans, des dendrocytes dermiques, des monocytes, des lymphocytes, des kératinocytes, des mastocytes et des cellules endothéliales.

Par « PKC » ou « Protéines Kinases C », on entend au sens de la présente invention les enzymes qui catalysent une réaction de phosphorylation sur un substrat cellulaire.

Lorsqu'elles sont activées, les PKC phosphorylent des résidus sérine ou thréonine spécifiques sur des substrats protéiques, qui varient selon le type cellulaire. Dans de nombreuses cellules, l'activation des PKC augmente la transcription de gènes spécifiques.

Les protéines kinases C (PKC) sont des protéines codées par une famille de gènes (11 isoformes différentes). On sait notamment que ces protéines sont impliquées dans la transduction de signaux extracellulaires médiés par les facteurs de croissance, les cytokines, ainsi que par un certain nombre d'autres molécules biologiques. La protéine kinase β2 (PKC-β2) apparaît exprimée spécifiquement par les CL de l'épiderme.

Tout composé connu de l'homme du métier comme inhibant l'activité de phosphorylation des PKC peut ainsi être utilisé en tant que composé inhibiteur des PKC selon la présente invention. On peut par exemple citer les polypeptides décrits dans la demande WO 99/43805 (Incyte Genomics Inc.).

En particulier, le composé inhibiteur des PKC est choisi dans le groupe constitué par les inhibiteurs non spécifiques des PKC, les inhibiteurs spécifiques de l'isoforme PKC-β2, et les associations de ceux-ci.

Plus particulièrement, le composé inhibiteur des PKC est choisi dans le groupe constitué par les composés phénoliques et polyphénoliques, les procyanidines (catéchines, épicatéchines, etc.), l'alpha-amyrine, le lupéol, le lupéol linoléate, les stérols, les stanols, les alcools triterpéniques et leurs homologues hydrogénés, les antibiotiques tels que la staurosporine, Ro-318425 (ou 2-(8)-(aminométhyl)-6,7,8,9-tétrahydropyridol(1,2-a)indol-3-yl)3-(1-méthyl-indol-3-ylmaléimide, HCl) tel que commercialisé par la société Calbiochem, les composés qui agissent par compétition avec les activateurs physiologiques des PKC tels que le diacylglycérol et le phorbol ester, les lipides cutanés de type (lyso)sphingolipides, lysophospholipides tels que les céramides et pseudocéramides, sphingosines et phytosphingosines, les sphinganines, les dérivés, précurseurs, analogues et homologues de ces composés, d'origine naturelle ou synthétique.

Par « composés phénoliques et polyphénoliques », on entend selon l'invention les phénols simples, les benzoquinones, les acides phénoliques, les acétophénones, les acides phénylacétiques, les acides hydroxycinnamiques, les coumarines et isocoumarines, les chromones, les naftoquinones, les xanthones, les anthraquinones, les flavonoides, les lignanes et néolignanes, les lignines, les chalcones, les dihydrochalcones, les aurones, les flavones, les flavonols, les dihydroflavonols, les flavanones, les flavanols, les flavandiols ou leucoanthocyanidines, les anthocyanidines, les isoflavonoides, les biflavonoides, les proanthocyanidines et les tanins condensés.

Par "stérols", on entend plus particulièrement selon l'invention le stérol, c'est à dire le composé perhydro-1,2-cyclopentanophenanthrène ayant un groupe hydroxyle à la position 3, et les analogues du stérol de formule générale (I) ci-dessous.

Ainsi, de préférence, les stérols utilisables selon l'invention répondent à la formule générale suivante : 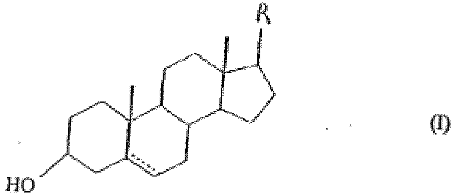 dans laquelle l'insaturation en pointillés en position 5 correspond à l'insaturation dans le cas des stérols, R représente une chaîne hydrocarbonée, linéaire ou ramifiée, insaturée ou non, comportant de 1 à 25 atomes de carbone. En particulier, R est choisi dans le groupe constitué par les groupes alkyle en C₁-C₁₂ les groupes alcoxy en C₁-C₈, les groupes alcényle en C₂-C₈, les groupes alcynyles en C2 - C8, les groupes cycloalkyle en C₃-C₈, les groupes alcényle en C₂-C₈ halogénés, les groupes alcynyles en C₂-C₈ halogénés. Le terme "halogéné" désigne un ou plusieurs substituant halogène, à savoir un ou plusieurs atome(s) de chlore, fluor, brome ou iode.

Parmi les stérols pouvant être avantageusement utilisés selon l'invention, on peut citer en particulier le β-sitostérol, l'α-sitostérol, le γ-sitostérol, le stigmastérol, le campestérol ou encore le brassicastérol et les mélanges de ceux-ci. Par exemple, le β-sitostérol peut être utilisé sous la forme du produit dénommé "Ultra" (comprenant principalement du β-sitostérol) tel que commercialisé par la société Kaukas. Dans le cas d'une utilisation d'un mélange de stérols, on peut citer par exemple le produit dénommé "Generol" comprenant principalement du β-sitostérol (environ 50 % en poids), du stigmastérol, du brassicastérol et du campestérol tel que commercialisé par la société Cognis ou encore le produit "Primal" de la société Kaukas.

Parmi les alcools triterpéniques pouvant être avantageusement utilisés selon l'invention, on peut citer en particulier la β-amyrine, l'érythrodiol, le taraxastérol, le cycloarténol, le 24-méthylènecycloartanol, le lupéol, le lanostérol et les mélanges de ceux-ci.

Par "homologues hydrogénés" d'un alcool triterpénique, on entend selon l'invention le ou les composés alcool(s) triterpénique(s) correspondant(s) dont la ou les liaison(s) insaturée(s) éventuellement présente(s) ont été hydrogénées (c'est à dire transformée(s) en liaison saturée) selon des méthodes bien connues de l'homme du métier.

Plus particulièrement encore, le composé inhibiteur des PKC est choisi dans le groupe constitué par les sphingolipides et les lysophospholipides, tels que :
- les céramides
- les sphingosines
- les galactocérobrosides
- les psychosines
- les sulfatides
- les lysosulfatides
- les sphyngomyélines, et
- les lysosphingomyélines.

On peut citer également plus particulièrement, comme composé inhibiteur des PKC, les lipides cutanés de type sphingolipides et lysophospholipides.

Comme sphingolipides, on peut citer ceux parmi les plus élémentaires tels que la sphingosine (D érythro dihydroxy 1,3 amino 2 octadécène 4t) et ses isomères, la phytosphingosine (D ribo trihydroxy 1,3,4 amino 2 octadécane) et ses isomères. Mais aussi, les lysosphingolipides (parmi eux, la lysosulfatide et la psychosine), la sulfogalactosylsphingosine, la sphinganine (2-amino 1,3 octadécane diol) et les sphingomyelines.

Comme phospholipides, on peut citer ceux parmi les familles des phosphatidylamino-alcools et des phosphatidylpolyols. Le groupe des phosphatidylamino-alcools comprend notamment les phosphatidylethanolamines (ou phosphatidylcolamines), les phosphatidylcholines, les phosphatidylsérines, les N-acylphosphatidylethanolamines. Celui des phosphatidylpolyols comprend quant à lui, les phosphatidylcholinositols, les diphospho-inositides, les lysodiphospho-inositides, les phosphatidylglycérols et les cardiolipides.

On peut citer également plus particulièrement, comme composé inhibiteur des PKC, les céramides, notamment les céramides du ciment intercornéocytaire de l'épiderme ainsi que les précurseurs des céramides que sont la sphingosine et la phytosphingosine.

D'une manière générale, les céramides peuvent être synthétisés chimiquement (on parle notamment de pseudo-céramides), être d'origine animale (des concentrations relativement élevées de sphingolipides sont présentes dans l'encéphale et la colonne vertébrale des mammifères), d'origine végétale (principalement des cérébrosides et autres sphingolipides glycosylées) ou encore être des dérivés de levures (configuration stéréochimique identique à celle des céramides naturellement présents dans la peau humaine).

Les céramides du ciment intercornéocytaire de l'épiderme peuvent être séparés par les méthodes classiques (chromatographie sur couche mince) en six fractions, correspondant à des composés différant par la nature des acides gras et la nature de la base impliquée (sphingosines, insaturées, ou phytosphingosines, saturées). Le tableau 1 suivant illustre les structures respectives présentes dans ces fractions, selon la classification de Werts et Downing. La fraction 6 peut elle-même être subdivisée par des méthodes plus fines en deux entités : les céramides 6a et 6b.

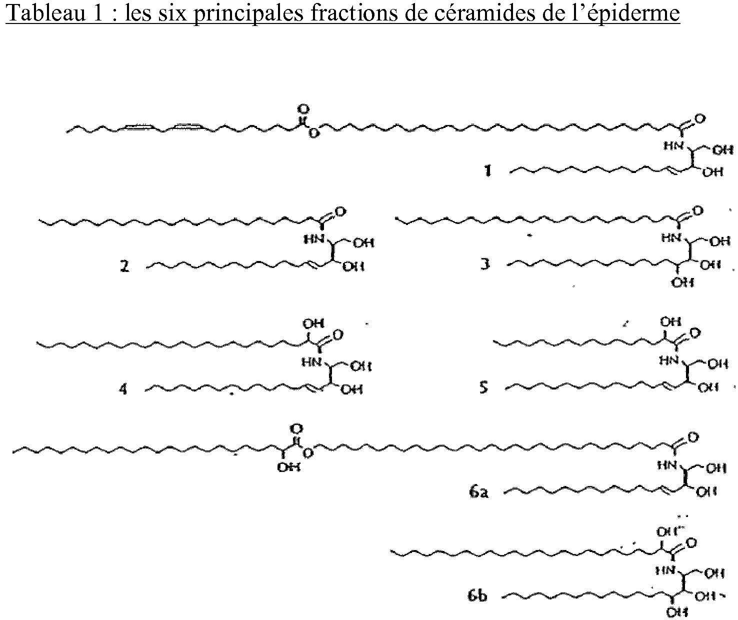

Ainsi, les céramides 1, les moins polaires, comportent une structure tout à fait particulière, que l'on retrouve dans le céramide 6a : un oméga-hydroxyacide à longue chaîne amidifiant la base, et attaché à son extrémité omega par une liaison ester à un autre acide gras (0-acylcéramides). Dans le cas de la fraction 1, les acides gras liés à la sphingosine sont essentiellement en C24, C26, C30, C32 ou C34, pouvant être saturés, monoéthyléniques (principalement pour les C30, C32 et C34) ou diéthyléniques (C32 et surtout C34). Quant à l'acide gras attaché à l'extrémité oméga du précédent, il s'agit, de façon largement prédominante pour les céramides 1, de l'acide linoléique, le rôle capital dans la fonction de barrière hybride de l'épiderme est bien connu.

La fraction 2, de structure plus classique (sphingosines ou dihydrosphingosines liées par une liaison amide à un acide gras, principalement C20 à C28), est la plus abondante.

La fraction 3 est assez similaire, la différence portant sur la nature de la base, qui, en l'occurrence, est essentiellement représentée par les phytosphingosines, saturées.

Les fractions 4 et 5 sont essentiellement caractérisées par la présence d'alpha-hydroxyacides liés à une sphingosine.

La fraction 6b est proche des fractions 4 et 5, comportant un alpha hydroxyacide, mais lié à une phytosphingosine, saturée.

La fraction 6a, comme le céramide 1, comporte le motif caractéristique que l'on ne retrouve qu'au niveau des céramides de l'épiderme, c'est-à-dire la liaison ester entre l'hydroxyle en oméga d'un acide gras lié à une sphingosine, et le groupement carboxylique d'un acide gras terminal, qui, cette fois, n'est pas l'acide linoléique, mais un alpha-hydroxyacide.

Il convient également de citer les phytocéramides (céramides à base phytosphingosine), les cholestérol-céramides synthétiques, les galacto ou gluco cérébrosides.

Enfin, parmi les composés inhibiteurs des PKC pouvant être utilisés selon la présente invention, la sphingosine est présente à l'état naturel dans la peau, et joue entre autre un rôle important dans fonction barrière du *stratum corneum,* en tant que précurseur des sphingolipides (céramides et sphingoglycolipides). Elle peut être dérivée de source biologique telle que des extraits de cerveaux bovins ou par voie de synthèse, à partir de la serine, comme décrit par exemple dans l'article de Newman, J.Am. CHEM., 95 (12) : 4098 (1973). On peut plus particulièrement citer les formes isomères de la sphingosine : D-érythro, L-thréo, L-érythro et D-thréo. La forme D-erythro est la plus fréquemment présente dans la nature.

Selon la présente invention, les composés inhibiteurs des PKC pouvant être utilisés comprennent les isomères, les dérivés (sels, complexes, etc.), les analogues, les homologues, les précurseurs et les métabolites des composés inhibiteurs des PKC décrits ci-dessus.

Les agents anti-inflammatoires pouvant être utilisés dans le cadre de la présente invention en association avec les alcanolamides sont avantageusement des agents anti-inflammatoires stéroïdiens (AIS), tels que les corticoïdes, ou non-stéroïdiens (AINS).

Les agents apaisants pouvant être utilisés dans le cadre de la présente invention en association avec les alcanolamides sont avantageusement des dérivés de réglisse et des dérivés de l'alpha-bisabolol.

Les immunosuppresseurs pouvant être utilisés dans le cadre de la présente invention en association avec les alcanolamides sont avantageusement le tacrolimus, le pimécrolimus, et la cyclosporine.

Les chélateurs d'ions pouvant être utilisés dans le cadre de la présente invention en association avec les alcanolamides sont avantageusement des chélateurs chimiques avantageusement choisis dans le groupe constitué par l'acide éthylènediamine-tétracétique (EDTA) et ses sels de sodium, de potassium, de calcium disodium, de diammonium, de triéthanololamine (TEA-EDTA), l'acide hydroxyéthyl éthylène diamine tétracétique (HEDTA) et son sel trisodique, l'acide diéthylènetriamine pentacétique (DTPA), et leurs mélanges. Les chélateurs d'ions peuvent également être des chélateurs biologiques avantageusement choisis dans le groupe constitué par la métallothionéine, la transférine, la lactoférine, la calmoduline, le chitosane méthylène phosphonate, et leurs mélanges.

Les ions chélatés sont avantageusement les ions Na⁺, K⁺, Ca²⁺, Cl⁻, Ni²⁺, Co⁺, Co²⁺, Zr²⁺, Zr⁴⁺, mais aussi les ions chrome au niveau d'oxydation II et III tels que Cr²⁺, Cr³⁺, et Cr₂O₇²⁻.

Les oxazolines pouvant être utilisées dans le cadre de la présente invention en association avec les alcanolamides sont avantageusement des oxazolines répondant aux formules générales suivantes: 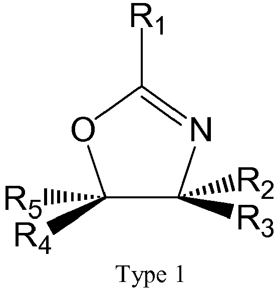 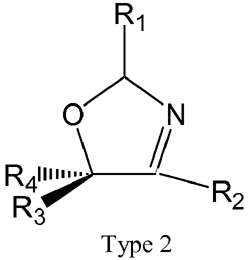 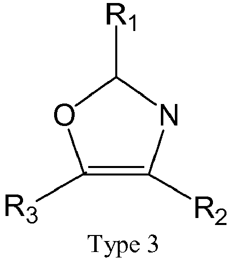 dans lesquelles R₁ représente un groupe alkyle en C₁-C₄₀, linéaire ou ramifié, saturé ou insaturé, comprenant éventuellement une ou plusieurs insaturation(s) éthylénique(s) ainsi que un ou plusieurs substituant(s) choisi(s) dans le groupe formé par les radicaux hydroxy (OH) et alcoxy en C₁-C₆ (OC₁-C₆) ; R₂, R₃, R₄ et R₅ représentent, de manière indépendante, un atome d'hydrogène, un radical hydroxy, ou un groupe alkyle en C₁-C₃₀, linéaire ou ramifié, saturé ou insaturé, comprenant éventuellement une ou plusieurs insaturations éthyléniques ainsi que un ou plusieurs substituant(s) choisi(s) dans le groupe formé par les radicaux hydroxy (OH), alcoxy en C₁-C₆ (OC₁-C₆) et alcoxy en C₁-C₆ carbonyles (COOC₁-C₆). Par le terme de "alcoxy en C₁-C₆ (OC₁-C₆)", on entend au sens de la présente invention, un radical alcoxy dont le groupement alkyle comprend de 1 à 6 atomes de carbone.

Avantageusement selon la présente invention, ladite oxazoline est une oxazoline de type 1 choisie dans le groupe constitué par la 2-undécyl-4-hydroxyméthyl-4-méthyl-1,3-oxazoline, la 2-undécyl-4,4-diméthyl-1,3-oxazoline, la (E)-4,4-diméthyl-2-heptadéc-8-ényl-1,3-oxazoline, la 4-hydroxyméthyl-4-méthyl-2-heptadécyl-1,3-oxazoline, la (E)-4-hydroxyméthyl-4-méthyl-2-heptadéc-8-ényl-1,3-oxazoline, la 2-undécyl-4-éthyl-4-hydroxyméthyl-1,3-oxazoline. De manière encore plus avantageuse, ladite oxazoline est la 2-undécyl-4,4-diméthyl-1,3-oxazoline, appelée OX-100, de formule :

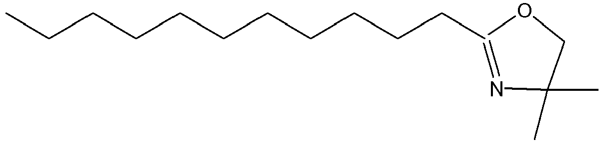

Les dérivés d'acide carbamique pouvant être utilisés dans le cadre de la présente invention en association avec les alcanolamides sont avantageusement des dérivés d'acide carbamique répondant aux formules générales suivantes: 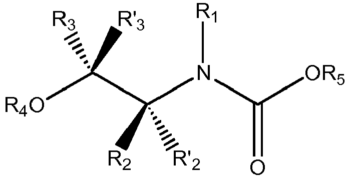 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₄ et R₅ représentent, de manière indépendante, un atome d'hydrogène ou un groupe acyle de type RxCO, dans lequel Rx est un radical alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation, R₁ représente un atome d'hydrogène.

Selon un autre mode de réalisation, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et/ou R'₂ représente un atome d'hydrogène.

Selon un autre mode de réalisation, R₃ et R'₃ représentent un atome d'hydrogène.

Selon un autre mode de réalisation, R₄ et R₅ représentent un atome d'hydrogène.

Dans un mode de réalisation particulier, R₁ représente un atome d'hydrogène, R₂ représente un groupe alkyle linéaire saturé en C₈-C₂₂, avantageusement en C₉-C₁₈, de manière encore plus avantageuse en C₉-C₁₃, de manière encore plus avantageuse en C₁₁-C₁₃, et R'₂, R₃, R'₃, R₄ et R₅ représentent un atome d'hydrogène.

Avantageusement le dérivé d'acide carbamique est choisi dans le groupe constitué par le (1-Hydroxyméthyl-tridécyl)-acide carbamique et le (1-Hydroxyméthyl-undécyl)-acide carbamique.

Le (1-Hydroxyméthyl-tridécyl)-acide carbamique peut être représenté par la formule suivante :

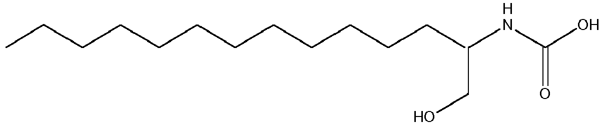

Les oxazolidinones pouvant être utilisées dans le cadre de la présente invention en association avec les alcanolamides sont avantageusement des oxazolidinones répondant aux formules générales suivantes: 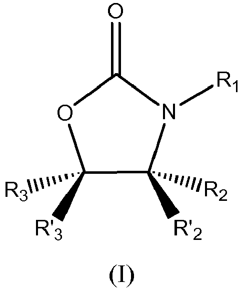 dans laquelle :
R₁ représente un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₂ et R'₂ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH);
R₃ et R'₃ représentent, de manière indépendante, un atome d'hydrogène ou un groupe alkyle en C₁-C₃₀, linéaire ou ramifié, saturé ou insaturé, comprenant éventuellement une ou plusieurs insaturation(s) éthylénique(s) et/ou acétylénique(s), ainsi que un ou plusieurs substituant(s) hydroxy (OH).

Selon un mode de réalisation, R₁ représente un atome d'hydrogène.

Selon un autre mode de réalisation, R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et/ou R'₂ représente un atome d'hydrogène.

Selon un autre mode de réalisation, R₃ et R'₃ représentent un atome d'hydrogène.

Dans un mode de réalisation particulier, R₁ représente un atome d'hydrogène, R₂ représente un groupe alkyle linéaire saturé en C₈-C₂₂, avantageusement en C₉-C₁₈, de manière encore plus avantageuse en C₉-C₁₂, de manière encore plus avantageuse en C₁₀-C₁₁, et R'₂, R₃ et R'₃ représentent un atome d'hydrogène.

Avantageusement l'oxazolidinone est choisie dans le groupe constitué par la 4-dodécyl-oxazolidin-2-one, la 3,4-didodécyl-oxazolidin-2-one et la 4,5-didodécyl-oxazolidin-2-one.

La 4-dodécyl-oxazolidin-2-one peut être représentée par la formule suivante :

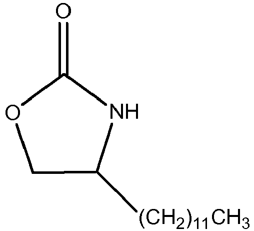

La 3,4-didodécyl-oxazolidin-2-one peut être représentée par la formule suivante :

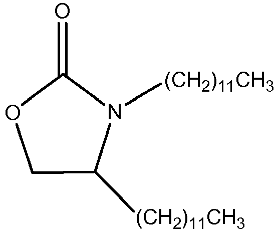

La 4,5-didodécyl-oxazolidin-2-one peut être représentée par la formule suivante :

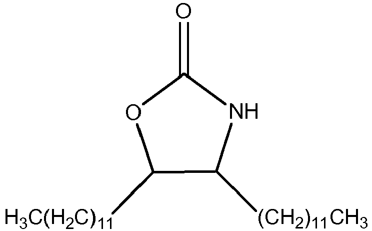

Les oxazolines, les oxazolidinones et les acides carbamiques sont des composés inhibiteurs de la migration des cellules de Langerhans.

La composition se caractérise en ce que la concentration en alcanolamide est avantageusement comprise entre environ 0,001 et environ 10 % en poids, et plus particulièrement entre environ 0,01 et 3 % en poids, par rapport au poids total de la composition.

La composition est avantageusement une composition cosmétique ou pharmaceutique, notamment dermatologique. La composition peut être formulée sous la forme de différentes préparations adaptées à une administration topique, à une administration orale ou rectale, à une administration parentérale. De préférence les différentes préparations sont adaptées à l'administration topique et incluent les crèmes, les pommades, les lotions, les huiles, les patches, les sprays ou tout autres produits pour application externe. Les modes d'administration, les posologies et les formes galéniques optimales des composés et compositions selon l'invention peuvent être déterminés selon les critères généralement pris en compte dans l'établissement d'un traitement cosmétique ou pharmaceutique, de préférence dermatologique, adapté à un patient comme par exemple l'âge ou le poids corporel du patient, la gravité de son état général, la tolérance au traitement, les effets secondaires constatés, le type de peau. En fonction du type d'administration souhaitée, la composition et/ou les composés actifs selon l'invention peuvent en outre comprendre au moins un excipient cosmétiquement acceptable ou pharmaceutiquement acceptable, notamment dermatologiquement acceptable. De préférence, on utilise un excipient adapté pour une administration par voie topique externe. La composition selon la présente invention peut en outre comprendre au moins un adjuvant cosmétiquement ou pharmaceutiquement connu de l'homme du métier, choisi parmi les épaississants, les conservateurs, les parfums, les colorants, des filtres chimiques ou minéraux, les agents hydratants, les eaux thermales, etc.

Ledit médicament est destiné au traitement ou à la prévention des réactions ou pathologies allergiques, et/ou inflammatoires, et/ou irritatives de la peau et/ou des muqueuses, notamment de la bouche, des poumons, de la vessie, du rectum, du vagin.

Il est ici décrit que le médicament est adapté au traitement et/ou à la prévention des réactions ou pathologies de la peau et/ou des muqueuses consécutives à la migration des cellules, telles que les cellules de Langerhans, induite par un signal danger. On entend par « signal danger »au sens de la présente invention tout signal entraînant notamment la production de cytokines inflammatoires ou tout signal immunologique vrai du type pénétration d'un allergène.

Selon un mode de réalisation de la présente invention, le médicament est destiné au traitement et/ou à la prévention des réactions ou pathologies induites par des haptènes chimiques ou métalliques.

Selon un autre mode de réalisation de la présente invention, le médicament est destiné au traitement ou à la prévention des peaux et/ou des muqueuses sensibles et/ou réactives et/ou inconfortable et/ou intolérante et/ou présentant un trouble de la barrière cutanée et/ou présentant un déséquilibre immunologique lié au vieillissement intrinsèque, extrinsèque (soleil, pollution) ou hormonal.

En effet, il a été montré que le vieillissement de la peau entraînait une modification du statut immunitaire de celle-ci et que les cellules immunologiques pouvaient modifier leur localisation initiale par suite d'une migration incontrôlée.

La composition selon l'invention, ainsi que les composés actifs selon l'invention permettent de réduire la réponse immunitaire induite par la migration de CL ayant fixé des IgE en surface. C'est pourquoi, la présente invention concerne également l'utilisation d'une composition selon l'invention et destinée à inhiber la migration des cellules de Langerhans ou d'au moins un composé actif choisi dans le groupe des alcanolamides telles que définies précédemment pour le traitement ou la prévention de l'eczéma atopique. La composition selon l'invention, ainsi que les composés actifs selon l'invention sont également destinés au traitement ou à la prévention de l'eczéma de contact, dans la mesure où ils permettent de réduire une réponse immunitaire notamment induite par capture d'un antigène, traitement et présentation de cet antigène aux lymphocytes T par les CL.

La composition, ainsi que les composés actifs, sont également utilisés pour le traitement et/ou la prévention de pathologies inflammatoires, notamment de dermatoses inflammatoires telles que le psoriasis, des dermites irritatives, de maladies auto-immunes, de la prévention la photo-immuno-suppression ou du rejet de greffe. Par « photo-immuno-suppression » au sens de la présente invention, on entend désigner une diminution de la réponse immunitaire induite par les ultra-violets solaires, et plus particulièrement par les ultra-violets B.

La composition, ainsi que les composés actifs, sont également utilisés pour diminuer le caractère allergisant et/ou irritant d'une composition, qui peut être une préparation pharmaceutique ou une préparation cosmétique, ou d'un parfum. Par caractère allergisant, on entend le pouvoir de certains composés contenus dans les dites compositions de se comporter comme des allergènes, c'est-à-dire des composés capables d'induire une réaction d'hypersensibilité immédiate et/ou inflammatoire.

Dans les différentes utilisations précédemment évoquées du composé actif choisi dans le groupe des alcanolamides tels que définis ci-dessus, celui-ci peut-être utilisé en association avec au moins un composé, inhibiteur de la migration des cellules de Langerhans, sélectionné dans le groupe constitué par les inhibiteurs de métalloprotéases matricielles (MMPs), les inhibiteurs de PKC, les agents anti-inflammatoires, les agents apaisants, les immunosuppresseurs, les chélateurs d'ions, les oxazolidinones, les dérivés d'acide carbamique et les oxazolines tels que définis précédemment.

La composition et les composés actifs sont avantageusement destinés à une utilisation en cosmétologie. La présente invention concerne également une composition telle que définie dans les revendications pour son utilisation d'une méthode de traitement des peaux et/ou des muqueuses sélectionnées parmi les peaux et/ou les muqueuses sensibles, irritées, intolérantes, à tendance allergique, âgées, soumises à un signal danger, présentant un trouble de la barrière cutanée, présentant des rougeurs cutanées, ou présentant un désordre ou un déséquilibre immunologique non pathologique lié au vieillissement intrinsèque, extrinsèque ou hormonal, caractérisée en ce qu'elle consiste à appliquer sur la peau et/ou les muqueuses une composition ou au moins un composé actif choisi dans le groupe des alcanolamides telles que définies précédemment. Le composé actif choisi dans le groupe des alcanolamides tels que définis précédemment peut également être appliqué en association avec au moins un autre composé sélectionné dans le groupe constitué par les inhibiteurs de métalloprotéases matricielles (MMPs), les inhibiteurs de PKC, les agents anti-inflammatoires, les agents apaisants, les immunosuppresseurs, les chélateurs d'ions, les oxazolidinones, les dérivés d'acide carbamique et les oxazolines tels que définis précédemment. Dans le cadre de la méthode de traitement cosmétique, le désordre ou le déséquilibre immunologique non pathologique est un déséquilibre temporaire ou non de la fonction immunitaire de la peau sans caractère de gravité.

D'autres caractéristiques et avantages de l'invention apparaissent dans la suite de la description avec les exemples représentés ci-après. Dans ces exemples on se référera aux figures suivantes. Ces figures et exemples sont destinés à illustrer la présente invention et ne peuvent en aucun cas être interprétés comme pouvant en limiter la portée.

### FIGURES

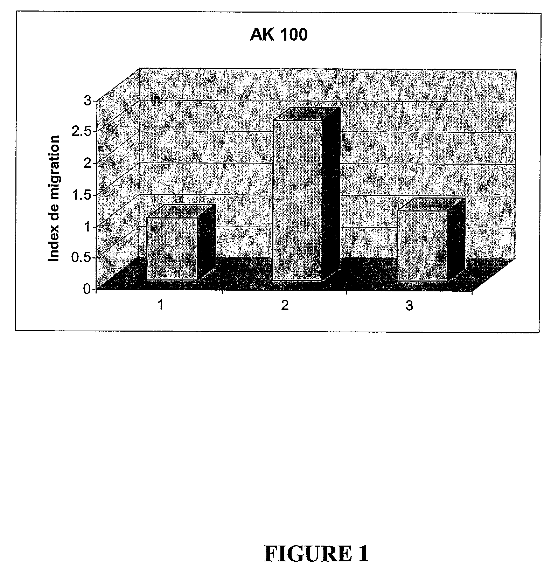

Figure N°1 : Index de migration de cellules de Langerhans fraîchement isolées à partir de peau humaine et activées par du DNSB. Effet de la molécule d'alcanolamide AK100 sur le phénomène migratoire. (1) cellules contrôles ; (2) cellules sensibilisées par l'haptène DNSB ; (3) cellules sensibilisées par l'haptène DNSB + AK100 (1 µM).

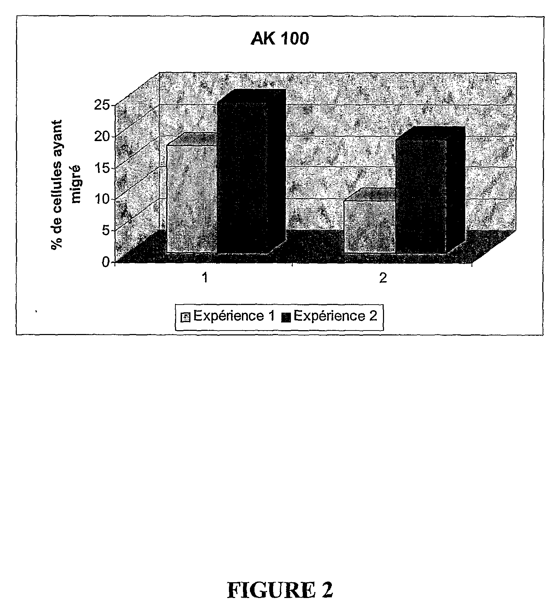

Figure N°2 : Pourcentage (%) de migration de cellules dendritiques issues de sang de cordon, après activation par l'haptène. Effet de la molécule d'alcanolamide AK100 sur le phénomène migratoire. (1) cellules sensibilisées par l'haptène BB ; (2) cellules sensibilisées par l'haptène BB + AK100 (1 µM).

### EXEMPLES

### Exemple 1 : Exemple de composition selon la présente invention

| | % en poids |
|---|---|
| Eau | qsp 100 |
| Polydécène hydrogéné | 5 à 8 |
| Glycérine | 4 à 17 |
| Carbonate de dicaprylyle | 4 à 9 |
| Glucoside de lauryle | 1 à 4,5 |
| Polyglycéryl-2 dipolyhydroxystéarate | 2 à 5 |
| Extrait peptidique de lupin (Protéine hydrolysée) | 0,1 à 3 |
| Acrylate/Copolymère d'acrylate d'alkyle en C₁₀₋₃₀ | 0,4 |
| Hydroxyméthylglycinate de sodium | 0,4 |
| Gomme de xanthane | 0,3 |
| Alcanolamide AK100 | 0,01 à 0,8 |
| Hydroxyde de sodium | 0,07 |
| Acide citrique | 0,03 |

### Exemple 2 : étude de l'activité de l'AK100 sur l'inhibition de la migration des CL fraîchement isolées à partir de fragments de peau humaine

### 1) Matériels & Méthodes

### 1.1. Obtention des cellules de Langerhans

Des suspensions de cellules épidermiques ont été obtenues par traitement enzymatique (0,05% trypsine, pendant 18h à +4°C) de fragments de peau humaine normale issus de chirurgie plastique. Les suspensions obtenues contiennent en moyenne 2 à 4% de CL. L'obtention de suspensions contenant en moyenne 70% de CL, est basée sur le principe de la centrifugation sur gradient de densité (Lymphoprep™) et élimination des kératinocytes.

### 1.2. Préparation des milieux

Le milieu de base choisi pour l'ensemble de l'étude a été le RPMI 1640 (Gibco BRL, France). La molécule AK100 fournie par Pharmascience, à la concentration de 10⁻² M en solution dans du DMSO (Dimethyl Sulfoxide), a été diluée en RPMI-1640 et testées à 1 µM.

### 1.3. Sensibilisation des CL

On a utilisé comme agent sensibilisateur le DNSB (Sigma Aldrich), forme soluble du DNCB (dinitro-chloro-benzène), solubilisé en RPMI-BSA et utilisé à la concentration de 50 µM.

### 1.4. Migration des CL

Un système de chambre de culture à deux compartiments (Falcon, Becton Dickinson, France) a été utilisé. Le compartiment supérieur est séparé du compartiment inférieur par une membrane de porosité 8 µm, sur laquelle sont déposées 50 µg/cm² de Matrigel. La membrane est alors recouverte de protéines formant un film équivalent à une membrane basale (laminine, collagène IV, nidogène, entactine, héparane sulfate protéoglycanes). Les cellules reprises dans le milieu RPMI-BSA seul ou en présence des différents produits sont déposées dans le compartiment supérieur. Dans le compartiment inférieur, est ajouté du surnageant de culture de fibroblastes humains normaux. Après 18h d'incubation à 37°C, le nombre de cellules vivantes ayant traversé le Matrigel et se trouvant dans le compartiment inférieur est compté sous microscope (les CL sont facilement identifiables par leur forme dendritique). Chaque essai est réalisé en triplicate.

### 2) Résultats

**2.1.** Les résultats sont présentés dans le tableau 1 suivant et illustrés par l'histogramme de la Figure 1.

**Tableau 2 : index de migration des CL**

| | 1 | 2 | 3 |
|---|---|---|---|
| Index de migration | 1 | 2,55 | 1,11 |

| | | | |
|---|---|---|---|
| *Légende tableau 2 et de l'histogramme de la* *figure 1* *:* 1 : Cellules contrôles 2 : Cellules sensibilisées par l'haptène DNSB 3 : DNSB + AK100 (1 µM) | | | |

### 2.2. Migration des CL

Les résultats représentent le rapport entre le nombre de cellules ayant migré en présence de DNSB +/- AK100 et le nombre de cellules ayant migré dans les conditions normales (cellules contrôles non sensibilisées et non traitées) . Les CL fraîchement isolées de l'épiderme n'ont pas une capacité migratoire élevée. Dans l'expression des résultats, la capacité migratoire des CL contrôles (non traitées et non sensibilisées) est arbitrairement fixée à 1.

Le traitement des cellules avec l'haptène DNSB a stimulé la migration des CL de façon significative (+155%) par rapport aux cellules normales non stimulées (cellules contrôles).

L'AK100 à la concentration de 1 µM inhibe de manière significative la migration des CL induite par le DNSB. Les cellules ainsi traitées ont un index de migration comparable à celui des cellules contrôles non sensibilisées.

Les inventeurs ont montré en utilisant des CL fraîchement isolées, placées dans un système de chambre de culture à deux compartiments (permettant la migration cellulaire), que de manière tout à fait surprenante, l'AK100 inhibe de manière significative la migration des CL. Dans les conditions expérimentales utilisées par les inventeurs, les cellules traitées par l'AK100 ont un index de migration comparable à celui des cellules contrôles non sensibilisées.

### Exemple 3: étude de l'activité de l'AK100 sur l'inhibition de la migration des cellules dendritiques générées in vitro à partir de précurseurs CD34+ issus de sang de cordon

### 1) Matériels & Méthodes

### 1.1.Génération de Langerhans-like in vitro

Les cellules mononuclées ont été obtenues à partir de sang de cordon ombilical de donneurs sains, par centrifugation sur Ficoll. Les cellules CD34+ ont ensuite été purifiées par immunosélection à l'aide d'anticorp spécifique et de billes magnétiques (Miltenyi Biotech, Germany). Les cellules CD34+ ont été cultivées en présence de GM-CSF (100 ng/ml), TNF-α (2.5 ng/ml) en RPMI additionné de 10% sérum de veau foetal, pendant 5 jours. L'addition de TGF-ß1, facteur qui favorise la différenciation des cellules vers la voie cellules de Langerhans, a été réalisée au 5ième jour de culture.

### 1.2.Préparation des milieux

Idem exemple 2.

### 1.3.Sensibilisation des CL

Les cellules ont été traitées, au 7ième jour par l'haptène BB (Base de Brandowski, 1.17 µg/ml), pendant 24 h, puis soumises au test de migration.

### 1.4.Migration des CL

Idem exemple 2.

### 2) Résultats

**2.1.** Les résultats de deux expériences indépendantes sont présentés dans le tableau 2 suivant et illustrés par l'histogramme de la Figure 2.

**Tableau 3 : Pourcentage de cellules dendritiques générées in vitro ayant migré**

| | 1 | 2 |
|---|---|---|
| Expérience 1 | 17 | 8,4 |
| Expérience 2 | 24 | 18 |

| | | |
|---|---|---|
| *Légende tableau 3 et de l'histogramme de la* *figure 1* 1 : Cellules sensibilisées par l'haptène BB 3 : BB + AK100 (1 µM) | | |

### 2.2. Migration des CL

Les résultats représentent le pourcentage de cellules ayant migré en présence des différents produits testés. Le pourcentage est calculé en rapportant le nombre de cellules récupéré dans le compartiment inférieur de la chambre de migration au nombre de cellules soumises à la migration. Dans les exemples 1 et 2, l'AK100 inhibe de respectivement 51 et 25% la migration des cellules dendritiques.

L'AK100 à la concentration de 1 µM inhibe de manière significative la migration des cellules dendritiques générées *in vitro* et activées par l'haptène BB.

### Exemple 4 : Etude de l'activité de l'alcanolamide AK100 seul ou en association avec LU105, sur l'inhibition de la migration des cellules dendritiques sur la souris

### 1) Matériels & méthodes

### 1.1. Réactifs

Le FITC (Fluorescéine isothiocyanate, Sigma, St. Louis, MO) a été dilué extemporanément dans un mélange acétone : dibutylphthalate (1:1).

### 1.2. Inhibiteurs

L'alcanolamide AK100 et le LU105 (LU 105 est un inhibiteur de MMPs, correspondant à un extrait peptidique de lupin blanc commercialisé par la Société Expanscience sous le nom de marque Actimp 193®) ont été fournis par les "Laboratoires Expanscience", et formulés seuls ou en association dans un véhicule inerte compatible avec une application topique, tel que décrit à l'exemple 1 ci-dessus [alcanolamide AK100 (0,1%)± LU105 (2%)]. Les différentes formulations ont été appliquées sur les oreilles de souris deux fois par jour pendant 4 jours consécutifs. Trois heures après la dernière application, 1,5% de FITC sont appliqués sur les deux oreilles (l'une traitée et l'autre non traitée (Contrôle)).

### 1.3. Migration des cellules de Langerhans (CL) et des cellules dendritiques CD sur la souris

L'effet des deux molécules a été évalué in vivo chez la souris. La peau des deux oreilles est badigeonnée avec 1,5% FITC (2X5 µl). 24 h après, les souris sont sacrifiées et une suspension cellulaire est préparée à partir des ganglions auriculaires et cervicaux (ganglions drainants, ci-après dénommés GL) ou à partir des ganglions popliteaux (ganglions non drainants, contrôle négatif). Les tissus ont été découpés et les cellules sont séparées par filtration (filtre 100 µM, Falcon; Becton Dickinson), puis lavés. Les cellules sont ensuite centrifugées 10 min à 600 x g (m x s⁻²) sur gradient de metrizamide (14,5% dans du RPMI 1640 ; 7,5% SVF). Les cellules de l'interface sont récupérées, rincées puis marquées avec un AC anti-CDS86 PE-conjugué, biot-MHC CLII mAbs plus streptavidine-Cya (PharMingen) et analysées par cytométrie en flux. Seules les cellules FITC+, PE+ et Cya+ sont comptabilisées car elles représentent la population de cellules ayant migré de la peau vers les GL suite à l'application de l'haptène.

### 2) Résultats

L'application topique du véhicule seul n'entraîne aucune modification du nombre de CD FITC+ au niveau des GL. Le véhicule est donc sans effet sur les capacités migratoire des CD.

Les résultats portant sur la migration des CD sont présentés dans le tableau 4 suivant.

**Tableau 4 :**

| | ALCANOLAMIDE AK100 (0,1%) | LU105 (2%) | ALCANOLAMIDE AK100 (0,1 %) + LU105 (2%) |
|---|---|---|---|
| Inhibition de la migration en % | 30 | 40 | 90 |

La migration des CD au niveau des GL, suite à l'application de l'haptène FITC, est inhibée dans des proportions comparables et sans différence significative par l'alcanolamide AK100 à la dose de 0,1% et le LU105 à la dose de 2%.

Lorsque les deux types de molécules sont associés, cette inhibition est presque totale. En conclusion, il a ainsi été montré de manière tout à fait surprenante, en utilisant un modèle de souris sensibilisées par l'haptène FITC, que l'alcanolamide AK100 inhibe de manière significative la migration des CD vers les GL. Par ailleurs, l'alcanolamide AK100 et le LU105 agissent en synergie pour inhiber la migration des CD chez la souris sensibilisée.

### Exemple 5 : Etude de l'activité de l'alcanolamide AK100, seul ou en association avec OX100, sur l'inhibition de la migration des cellules dendritiques sur la souris

### Matériels & méthodes

### Réactifs

Le FITC (Fluorescéine isothiocyanate, Sigma, St. Louis, MO) a été dilué extemporanément dans un mélange acétone : dibutylphthalate (1:1).

### Inhibiteurs

L'alcanolamide AK100 et OX100 (décrit précédemment) ont été fournis par les « Laboratoires Expanscience », et formulés seuls ou en association dans un véhicule inerte compatible avec une application topique, [AK100 (0,05%) ± OX100 (0,05%)]. Les différentes formulations ont été appliquées sur les oreilles des souris deux fois par jour pendant 4 jours consécutifs. Trois heures après la dernière application, 1,5% de FITC sont appliquées sur les deux oreilles (l'une traitée et l'autre non traitée (Contrôle)).

### Migration des cellules de Langerhans (CL) et des cellules dendritiques CD sur la souris

L'effet des deux molécules a été évalué in vivo chez la souris. La peau des deux oreilles est badigeonnée avec 1,5% FITC (2X5 µl). 24 h après, les souris sont sacrifiées et une suspension cellulaire est préparée à partir des ganglions auriculaires et cervicaux (ganglions drainants, ci-après dénommés GL) ou à partir des ganglions popliteaux (ganglions non drainants, contrôle négatif). Les tissus ont été découpés et les cellules sont séparées par filtration (filtre 100 µM, Falcon ; Becton Dickinson), puis lavés. Les cellules sont ensuite centrifugées 10 min à 600 x g (m x s⁻²) sur gradient de metrizamide (14,5% dans du RPMI 1640 ; 7,5% SVF). Les cellules de l'interface sont récupérées, rincées puis marquées avec un AC anti-CDS86 PE-conjugué, biot-MHC CLII mAbs plus streptavidine-Cya (PharMingen) et analysées par cytométrie en flux. Seules les cellules ayant migré de la peau vers les GL suite à l'application de l'haptène.

### Résultats

L'application topique du véhicule seul n'entraîne aucune modification du nombre de CD FITC+ au niveau des GL. Le véhicule est donc sans effet sur les capacités migratoires des CD.

Les résultats portant sur la migration des CD sont présentés dans le tableau 5 suivant.

**Tableau 5 :**

| | AK100 (0,05%) | OX100 (0,05%) | AK100 (0,05%) + OX100 (0,05%) |
|---|---|---|---|
| Inhibition de la migration en % | 15 | 15 | 40 |

La migration des CD au niveau des GL, suite à l'application de l'haptène FITC, est inhibée dans des proportions comparables et sans différence significative par AK100 à la dose de 0,05% et OX100 à la dose de 0,05%.

Lorsque les deux types de molécules sont associés, cette inhibition est plus importante. En conclusion, il a ainsi été montré de manière tout à fait surprenante, en utilisant un modèle de souris sensibilisées par l'haptène FITC, que l'alcanolamide AK100 inhibe de manière significative la migration des CD vers les GL. Par ailleurs, l'alcanolamide AK100 et OX100 agissent en synergie pour inhiber la migration des CD chez la souris sensibilisée.

### Exemple 6: Evaluation des effets d'une crème cosmétique de jour comprenant l'alcalonamide AK100 pour peau hypersensible, irritée ou à prédisposition allergique.

Une crème de jour cosmétique comprenant 0,1% en poids d'AK100 et 2% en poids d'extrait peptidique de lupin blanc, LU105, par rapport au poids total de la crème, a été testée sur des sujets humains volontaires, avec la collaboration de médecins dermatologues.

Les buts principaux sont d'évaluer l'efficacité clinique et l'acceptabilité cosmétique de ladite crème de jour dans le contexte d'une utilisation normale du produit.

Le produit du test, mis à la disposition du praticien avec les informations publiques nécessaires, a été proposé par le Dermatologue à son patient en précisant des modalités d'applications quotidiennes suffisantes soit au moins 2 applications par jour. Le produit a été appliqué sur le visage matin et soir sur une peau propre et sèche.

Au total, la durée de l'étude pour chaque sujet a été de 4 semaines avec deux observations, relevées avant puis après cette période de 4 semaines d'application.

A la consultation finale, le sujet a été interrogé de façon non directive sur la survenue éventuelle d'effets indésirables locaux.

Les tests, suivant le protocole décrit précédemment, ont été effectués sur 37 femmes.

Les résultats sont évalués sur une échelle allant de 0 à 9. Une cotation de 0 correspond à une évolution de la peau, avant et après traitement, nulle, une cotation allant de 1 à 3 correspond à une évolution de la peau, avant et après le traitement, légère, une cotation allant de 4 à 6 correspond à une évolution de la peau, avant et après traitement, modérée, et une cotation allant de 7 à 9 correspond à une évolution de la peau, avant et après traitement, importante.

**Tableau 6 : Evaluation clinique du dermatologue sur les femmes à peaux sensibles et/ou irritées :**

| | Erythème | Sécheresse | Desquamation | OEdème | Vésicules | Rugosité | Prurit | Picotements | Sensation de brûlure | Douleur |
|---|---|---|---|---|---|---|---|---|---|---|
| Note moyenne sur 10 à J0 | 4,24 | 4,84 | 2,95 | 0,70 | 0,16 | 1,68 | 3,16 | 2,95 | 3,49 | 0,89 |
| Note moyenne sur 10 à J30 | 1,68 | 1,68 | 0,89 | 0,08 | 0,00 | 0,32 | 0,70 | 0,57 | 0,43 | 0,11 |
| % *évolution de J0* à *J30* | -61% | -65% | -70% | -88% | -100% | -81% | -78% | -81% | -88% | -88% |

**Tableau 7 : Evaluation clinique du dermatologue sur les femmes à peaux sensibles :**

| | Erythème | Sécheresse | Desquamation | OEdème | Vésicules | Rugosité | Prurit | Picotements | Sensation de brûlure | Douleur |
|---|---|---|---|---|---|---|---|---|---|---|
| Note moyenne sur 10 à J0 | 3,90 | 5,00 | 2,35 | 0,55 | 0,25 | 1,60 | 2,70 | 3,35 | 4,15 | 0,55 |
| Note moyenne sur 10 à J30 | 1,75 | 1,65 | 0,50 | 0,00 | 0,00 | 0,40 | 0,30 | 0,55 | 0,40 | 0,05 |
| % *évolution de J0 à J30* | -55% | -67% | -79% | -100% | -100% | -75% | -89% | -84% | -90% | -91% |

**Tableau 8 : Evaluation clinique du dermatologue sur les femmes à peaux irritées :**

| | Erythème | Sécheresse | Desquamation | OEdème | Vésicules | Rugosité | Prurit | Picotements | Sensation de brûlure | Douleur |
|---|---|---|---|---|---|---|---|---|---|---|
| Note moyenne sur 10 à J0 | 4,65 | 4,65 | 3,65 | 0,88 | 0,06 | 1,76 | 3,71 | 2,47 | 2,71 | 1,29 |
| Note moyenne sur 10 à J30 | 1,59 | 1,71 | 1,35 | 0,18 | 0,00 | 0,24 | 1,18 | 0,59 | 0,47 | 0,18 |
| % *évolution de J0* à *J30* | -66% | -63% | -63% | -80% | -100% | -87% | -68% | -76% | -83% | -86% |

Ces différents résultats montrent que ladite crème de jour améliore sensiblement l'état clinique des peaux hypersensibles, irritées ou à prédisposition allergique. Ces données sont confirmées par les volontaires qui sont satisfaits à 95% de la crème qui procure une sensation immédiate de confort (100%), atténue les réactions aux agressions de la pollution et au climat (62%), calme les irritations (88%), protège et apaise (80%), augmente le seuil de tolérance (76%).

Ladite crème, formulée sans parfum ni colorant, assure ainsi une hydratation efficace des couches supérieures de l'épiderme et apporte une réponse adaptée aux peaux hypersensibles, irritées ou à prédisposition allergique.

En conclusion, il a ainsi été montré de manière tout à fait surprenante, que l'AK100 en association avec LU105 inhibe presque totalement la migration des CD vers les GL. Par ailleurs, AK100 et LU105 agissent en synergie pour inhiber la migration des CD chez la souris sensibilisée.

Il a ainsi été montré de manière tout à fait surprenante, que les mêmes proportions en AK100 et en LU105, incorporées dans une crème cosmétique, permettent d'assurer une hydratation efficace des couches supérieures de l'épiderme et d'apporter une réponse adaptée aux peaux hypersensibles, irritées ou à prédisposition allergique.

## Revendications

1. Utilisation d'une composition comprenant un excipient adapté pour une administration par voie topique externe et au moins un composé actif pour inhiber la migration des cellules de Langerhans choisi dans le groupe des alcanolamides répondant aux formules générales suivantes: 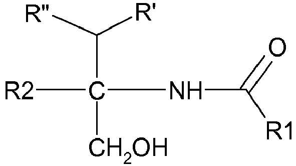
dans laquelle R₁ représente un groupe alkyle linéaire saturé en C₂-C₄₀;
R' et R" représentent, de manière indépendante, un atome d'hydrogène, un groupe méthyle ou un groupe alkyle linéaire saturé en C₂-C₂₀;
R₂ représente un atome d'hydrogène, un groupe méthyle ou un groupe alkyle linéaire saturé en C₂-C₂₀ ;
pour l'obtention d'un médicament destiné au traitement ou à la prévention des réactions ou pathologies de la peau et/ou des muqueuses consécutives à une migration des cellules de Langerhans choisies dans le groupe constitué par :
- les réactions ou pathologies allergiques ;
- les réactions ou pathologies inflammatoires ;
- l'eczéma atopique et/ou de contact ;
- les dermatoses inflammatoires telles que le psoriasis ;
- les dermites irritatives ;
- les maladies auto-immunes ;
- la photo-immuno-supression ; et
- le rejet de greffe.

2. Utilisation selon la revendication 1, **caractérisée en ce que** R₁ représente un groupe alkyle linéaire saturé en en C₆-C₂₂, avantageusement en C₈-C₁₈ et encore plus avantageusement en C₁₀-C₁₆.

3. Utilisation selon la revendication 1 ou 2, **caractérisée en ce que** ledit alcanolamide est l'alcanolamide appelé AK100 de formule :

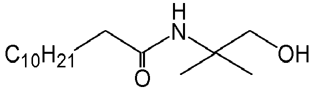

4. Utilisation selon l'une quelconque des revendications 1 à 3, **caractérisée en ce qu'**elle comprend en outre au moins un inhibiteur de métalloprotéases choisi dans le groupe constitué par les extraits peptidiques de lupin, de préférence l'extrait B (LU105), ou les rétinoïdes.

5. Utilisation selon l'une quelconque des revendications précédentes, **caractérisée en ce que** la concentration en alcanolamide dans la composition est comprise entre environ 0,001 et environ 10 % en poids, par rapport au poids total de la composition.

6. Utilisation selon l'une quelconque des revendications précédentes, **caractérisée en ce que** le médicament est destiné au traitement ou à la prévention des réactions ou pathologies induites par des haptènes chimiques ou métalliques.

7. Utilisation selon l'une quelconque des revendications 1 à 5, **caractérisée en ce que** le médicament est destiné à diminuer le caractère allergisant et/ou irritant d'une composition ou d'un parfum.

8. Composition telle que définie à l'une quelconque des revendications 1 à 5 pour son utilisation dans une méthode de traitement des peaux et/ou des muqueuses sensibles, irritées, intolérantes, à tendance allergique, soumises à un signal danger, présentant un trouble de la barrière cutanée, présentant des rougeurs cutanées, **caractérisée en ce qu'**elle est appliquée sur la peau et/ou les muqueuses.

## Patentansprüche

1. Verwendung einer Zusammensetzung, umfassend einen für eine Verabreichung auf externem topischem Weg geeigneten Arzneiträger und mindestens eine aktive Verbindung, um die Migration des Langerhans-Zellen zu hemmen, ausgewählt aus der Gruppe der Alcanolamide, die den folgenden allgemeinen Formeln entsprechen: 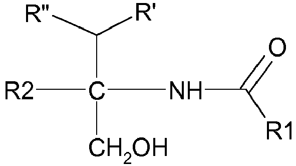
wobei R₁ eine gesättigte lineare C₂-C₄₀-Alkylgruppe darstellt;
R' und R" unabhängig ein Wasserstoffatom, eine Methylgruppe oder eine gesättigte lineare C₂-C₂₀-Alkylgruppe darstellen,
R₂ ein Wasserstoffatom, eine Methylgruppe oder eine gesättigte lineare C₂-C₂₀-Alkylgruppe darstellt,
um ein Arzneimittel zu erhalten, das für die Behandlung oder Prävention von Reaktionen oder Pathologien der Haut und/oder der Schleimhäute nach einer Migration der Langerhans-Zellen bestimmt ist, ausgewählt aus der Gruppe, die gebildet ist von:
- den allergischen Reaktionen oder Pathologien,
- den entzündlichen Reaktionen oder Pathologien,
- dem atopischen und/oder Kontaktekzem,
- den entzündlichen Dermatosen wie die Psoriasis,
- den irritativen Dermatitiden,
- den Autoimmunkrankheiten,
- der Photo-Immunsuppression und
- der Transplantatabstoßung.

2. Verwendung nach Anspruch 1, **dadurch gekennzeichnet, dass** R₁ eine gesättigte lineare C₆-C₂₂-, in vorteilhafter Weise C₆-C₁₈- und noch vorteilhafter C₁₀-C₁₆-Alkylgruppe darstellt.

3. Verwendung nach Anspruch 1 oder 2, **dadurch gekennzeichnet, dass** das Alcanolamid das Alcanolamid mit der Bezeichnung AK100 ist der Formel:

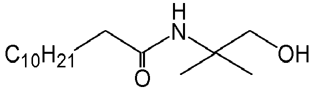

4. Verwendung nach einem der Ansprüche 1 bis 3, **dadurch gekennzeichnet, dass** sie ferner mindestens einen Metallproteasenhemmer umfasst, der aus der Gruppe ausgewählt ist, die von den Peptidextrakten der Lupine, vorzugsweise vom Extrakt B (LU105), oder den Retinoiden gebildet ist.

5. Verwendung nach einem der vorangehenden Ansprüche, **dadurch gekennzeichnet, dass** die Alkanolamidkonzentration in der Zusammensetzung zwischen zirka 0,001 und zirka 10 Gew.-% inklusive in Bezug zum Gesamtgewicht der Zusammensetzung beträgt.

6. Verwendung nach einem der vorangehenden Ansprüche, **dadurch gekennzeichnet, dass** das Arzneimittel für die Behandlung oder die Prävention der Reaktionen oder Pathologien bestimmt ist, die von chemischen oder metallischen Haptenen induziert werden.

7. Verwendung nach einem der Ansprüche 1 bis 5, **dadurch gekennzeichnet, dass** das Arzneimittel bestimmt ist, den allergieauslösenden und/oder irritierenden Charakter einer Zusammensetzung oder eines Duftstoffs zu verringern.

8. Zusammensetzung, wie nach einem der Ansprüche 1 bis 5 definiert, für ihre Verwendung in einer Behandlungsmethode der sensiblen, irritierten, intoleranten, eher allergischen Haut und/oder der Schleimhäute, die einem Gefahrensignal ausgesetzt sind, eine Störung der Hautbarriere aufweisen, Hautrötungen aufweisen, **dadurch gekennzeichnet, dass** sie auf die Haut und/oder die Schleimhäute aufgetragen wird.

## Claims

1. Use of a composition comprising an excipient adapted for external topical administration and at least one active compound to inhibit migration of Langerhans cells selected from the group of alkanolamides satisfying the following general formula: 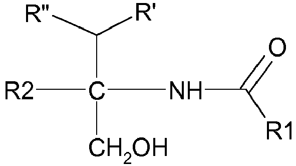
in which R₁ represents a C₂-C₄₀ saturated linear alkyl group;
R' and R" represent, independently, a hydrogen atom, a methyl group or a C₂-C₂₀ saturated linear alkyl group;
R₂ represents a hydrogen atom, a methyl group or a C₂-C₂₀ saturated linear alkyl group;
in the manufacture of a medicinal product intended for the treatment or prevention of reactions or pathologies of the skin and/or mucosa following migration of Langerhans cells selected from the group consisting of:
- allergic reactions or pathologies;
- inflammatory reactions or pathologies;
- atopic and/or contact eczema;
- inflammatory dermatoses such as psoriasis;
- irritative dermatitis;
- autoimmune diseases;
- photo-immunosuppression; and
- graft rejection.

2. Use according to claim 1, **characterized in that** R₁ represents a C₆-C₂₂, advantageously a C₈-C₁₈, and even more advantageously a C₁₀-C₁₆, saturated linear alkyl group.

3. Use according to claim 1 or 2, **characterized in that** said alkanolamide is the alkanolamide called AK100 of formula:

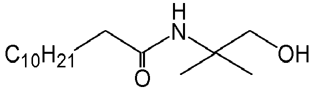

4. Use according to any one of claims 1 to 3, **characterized in that** it further comprises at least one metalloprotease inhibitor selected from the group consisting of peptide extracts of lupin, preferably extract B (LU105), or retinoids.

5. Use according to any one of the preceding claims, **characterized in that** the alkanolamide concentration in the composition is between about 0.001% and about 10% by weight, compared to the total weight of the composition.

6. Use according to any one of the preceding claims, **characterized in that** the medicinal product is intended for the treatment or prevention of reactions or pathologies induced by chemical or metallic haptens.

7. Use according to any one of claims 1 to 5, **characterized in that** the medicinal product is intended to diminish the allergenic and/or irritant nature of a composition or a fragrance.

8. Composition as defined in any one of claims 1 to 5 for use in a method for treating sensitive, irritated, intolerant or allergy-prone skin and/or mucosa, to which a danger signal is applied, exhibiting a skin barrier disorder, with skin rashes, **characterized in that** it is applied to the skin and/or mucosa.
